# EUROPÄISCHE PATENTSCHRIFT

(11) **EP 2 198 058 B1**
(45) Veröffentlichungstag und Bekanntmachung des Hinweises auf die Patenterteilung: **05.08.2015**
(21) Anmeldenummer: 08838200.7
(22) Anmeldetag: 01.10.2008
(51) Int. Cl.: C12Q 1/68, A61K 31/7088, G01N 33/574

(54) **MARKER ZUR DIAGNOSE VON KREBS**
MARKER FOR THE DIAGNOSIS OF CANCER
MARQUEUR POUR LE DIAGNOSTIC DU CANCER

(30) Priorität: 02.10.2007 DE 102007048636
(43) Veröffentlichungstag der Anmeldung: 23.06.2010
(73) Patentinhaber: Eberhard-Karls-Universität Tübingen, 72076 Tübingen (DE)
(72) Erfinder: ZIEKER, Derek, 72072 Tuebingen (DE)
(74) Vertreter: Witte, Weller & Partner Patentanwälte mbB
(86) Internationale Anmeldenummer: PCT/EP2008/008322
(87) Internationale Veröffentlichungsnummer: WO 2009/046917

(56) Entgegenhaltungen:
- WO-A-2005/001126
- WO-A-2007/065010
- ZHANG DAOHAI ET AL: "Proteomic study reveals that proteins involved in metabolic and detoxification pathways are highly expressed in HER-2/neu-positive breast cancer." MOLECULAR & CELLULAR PROTEOMICS : MCP NOV 2005, Bd. 4, Nr. 11, November 2005 (2005-11), Seiten 1686-1696, XP002511246 ISSN: 1535-9476
- WANG JIANHUA ET AL: "A glycolytic mechanism regulating an angiogenic switch in prostate cancer" CANCER RESEARCH, Bd. 67, Nr. 1, Januar 2007 (2007-01), Seiten 149-159, XP002511247 ISSN: 0008-5472
- ROSE PETER G ET AL: "Gemcitabine reverses cisplatin resistance: Demonstration of activity in platinum- and multidrug-resistant ovarian and peritoneal carcinoma." GYNECOLOGIC ONCOLOGY, Bd. 88, Nr. 1, Januar 2003 (2003-01), Seiten 17-21, XP002511248 ISSN: 0090-8258
- DE LANGE S M ET AL: "Phase II trial of cisplatin and gemcitabine in patients with advanced gastric cancer." ANNALS OF ONCOLOGY : OFFICIAL JOURNAL OF THE EUROPEAN SOCIETY FOR MEDICAL ONCOLOGY / ESMO MAR 2004, Bd. 15, Nr. 3, März 2004 (2004-03), Seiten 484-488, XP002511249 ISSN: 0923-7534
- KRISHNAN PREETHI ET AL: "Phosphorylation of pyrimidine deoxynucleoside analog diphosphates: selective phosphorylation of L-nucleoside analog diphosphates by 3-phosphoglycerate kinase." THE JOURNAL OF BIOLOGICAL CHEMISTRY 15 FEB 2002, Bd. 277, Nr. 7, 15. Februar 2002 (2002-02-15), Seiten 5453-5459, XP002511250 ISSN: 0021-9258
- SUN X -M ET AL: "Detection of type IV collagenase activity in malignant ascites" WORLD JOURNAL OF GASTROENTEROLOGY 200311 CN, Bd. 9, Nr. 11, November 2003 (2003-11), Seiten 2592-2595, XP002511276 ISSN: 1007-9327
- ZIEKER DEREK ET AL: "PGK1 a potential marker for peritoneal dissemination in gastric cancer." CELLULAR PHYSIOLOGY AND BIOCHEMISTRY : INTERNATIONAL JOURNAL OF EXPERIMENTAL CELLULAR PHYSIOLOGY, BIOCHEMISTRY, AND PHARMACOLOGY 2008, Bd. 21, Nr. 5-6, 2008, Seiten 429-436, XP002511251 ISSN: 1421-9778
- Stefan Kress ET AL: "Expression of hypoxia-inducible genes in tumor cells", Journal of Cancer Research and Clinical Oncology, vol. 124, no. 6, 1 January 1998 (1998-01-01), pages 315-320, XP055054357, ISSN: 0171-5216, DOI: 10.1007/s004320050175

## Beschreibung

Die vorliegende Erfindung betrifft allgemein die Verwendung von Markern zur Diagnose oder Vorhersage von Peritonealkarzinosen, sowie Verfahren zum Nachweis solcher Krankheiten in biologischen Proben, die Verwendungen der Marker als Targets für eine therapeutische Behandlung dieser Krankheiten und diesbezügliche pharmazeutische Zusammensetzungen und Diagnosekits.

Tumoren stellen im Allgemeinen Gewebswucherungen dar, die als Folge krankhafter übermäßiger Zellvermehrung entstehen. Tumorzellen, bzw. Krebszellen, sind Zellen, die - bspw. durch Mutationen - genetisch verändert werden und die aufgrund uneingeschränkter Teilung und aufgrund der Fähigkeit, sich über Lymphgefäße und Blutgefäße zu verbreiten und somit in anderen Geweben anzusiedeln, zur Bildung von Tumoren führen.

So zeichnen sich Tumorzellen meist neben einer veränderten Morphologie auch durch eine Zellkernpolymorphie aus und neigen zu Fokusbildung. Dies bedeutet, dass sie nicht mehr kontaktinhibiert sind, so dass es zu Verwachsungen kommen kann. Darüber hinaus verhalten sich Tumorzellen vielfach nicht mehr funktionsgerecht können oftmals durch De-Differenzierung ihre ursprünglichen Aufgaben nicht mehr wahrnehmen. Ferner sind in den Zellmembranen von Tumorzellen neue, sog. Tumor-Antigene oder Tumor-assoziierte Antigene, zu finden, die für die Diagnose von Tumoren als Marker eingesetzt werden können.

So ist bspw. das veränderte Verhalten der Tumorzellen und die Präsentation von Tumor-assoziierten Antigenen in der Zellmembran auf Veränderungen in der Genexpression sowie im Stoffwechsel der Tumorzellen zurückzuführen, was sich auch auf eine veränderte Signaltransduktion auswirkt. Mit diesem veränderten Verhaltensmuster und Expressionsmuster ist andererseits auch das Auftreten von für die Tumorzellen spezifischen Markern verbunden, mit deren Hilfe sich das Vorliegen von Tumoren im Körper eines Patienten nachweisen lässt.

Ein wichtiger Aspekt von malignen Tumoren ist ferner die Bildung von Metastasen, auch als Metastasierung bezeichnet. Bei diesem Vorgang gelangen Tumorzellen von ihrer primären Lokalisierung, als dem sog. Primärtumor, durch Blut- oder Lymphgefäße in primär nicht erkrankte Organe und Gewebe, wo es dann durch die Ansiedlung dieser Zellen zur Ausbildung von Metastasen, d.h. Tochtergeschwülsten, kommt. Durch diese Invasion können sich Tumorzellen im Körper verbreiten, und sogar vom Primärtumor weit entfernte Gewebe besiedeln, wo dann wiederum neue Tumore gebildet werden können.

Die Entstehung von maglinen Tumoren, d.h. die Karzinogenese, lässt sich in drei Phasen einteilen: So wird in der Initialphase die Zelle irreversibel transformiert, und zwar bspw. durch die Aktivierung von Proto-Onkogenen, durch karzinogene Hormone, onkogene Viren, genetisch bedingte Defekte der Systeme der DNA-Reparatur, Mutationen, erworbene oder angeborene Immundefekte, Strahlungen oder Karzinogene. In der darauf folgenden Latenzphase proliferieren die transformierten Zellen durch weiteres Einwirken von Karzinogenen oder anderen Faktoren, und zum Teil kann es bereits in dieser Phase zur Bildung von Metastasen kommen. Schließlich entstehen in der Manifestationsphase der Tumorbildung aus Frühformen oder auch aus zunächst gutartigen Tumoren maligne Formen, die dazu neigen, infiltrierend zu wachsen und Metastasen zu bilden.

Generell ist eine Früherkennung und die Diagnose von Tumoren, insbesondere metastasierenden Tumoren von großer Wichtigkeit, damit noch möglichst frühzeitig und rasch Therapien angesetzt werden können. Je früher der Tumor erkannt wird, desto besser ist die Prognose für eine Heilung bzw. erfolgreiche Behandlung. Gegenwärtig werden eine Reihe von Tumormarkern klinisch eingesetzt, die, wie weiter oben dargestellt, bspw. Substanzen und/oder zelluläre Veränderungen darstellen, durch deren qualitative und/oder quantitative Analyse ein Hinweis über das Vorliegen, den Verlauf oder die Prognose von malignen Tumoren gewonnen werden kann. Solche Tumormarker können, wie zuvor erwähnt, membranständige Tumor-Antigene sein, ferner Rezeptoren und Zellmarker, die auf eine vermehrte Expression von Onkogenen und ein monoklonales Zellwachstum hindeuten. Darüber hinaus kommen auch Substanzen für die Tumordiagnose in Frage, die im Vergleich zu Proben eines gesunden Patienten, in Körperproben von erkrankten Patienten, bspw. im Serum, Urin und/oder anderen Körperflüssigkeiten oder in Geweben, in erhöhten Konzentrationen auftreten. Solche Substanzen werden vom Tumorgewebe synthetisiert und/oder sezerniert, durch Tumorzerfall freigesetzt oder als Reaktion des Organismus auf einen Tumor gebildet.

Zu den in den meisten Fällen tödlich verlaufenden Tumorarten zählen u.a. Peritonealkarzinosen, die sich häufig bei Patienten entwickeln, die an Magentumoren leiden, und die häufig nach chirurgischen Eingriffen auftreten. Peritonealkarzinosen bilden sich dadurch, dass Magentumoren Krebszellen in die Bauchhöhle disseminieren, d.h. es handelt sich bei solchen Magentumoren um metastasierende Primärtumoren. Bis heute gibt es praktisch kein Heilmittel für diese Tumorerkrankung, was insbesondere in Anbetracht der äußerst geringen Überlebenschance (5 %) von Patienten mit Peritonealkarzinosen dramatisch ist.

Auch bei vielen weiteren, metastasierenden Primärtumoren, insbesondere des Gastrointestinaltraktes, gibt es bis heute keine Marker, anhand derer eine Metastasenbildung des Primärtumors vorhergesagt werden könnte. Zu dem Gastrointestinaltrakt zählen Speiseröhre Magen, Dünndarm, Dickdarm (Kolon), und Bauchspeicheldrüse (Pankreas), sowie Zwölffingerdarm (Duodenum), Leerdarm (Jejunum) und Krummdarm (Illeum), in welche sich der Dünndarm weiter unterteilen lässt. Die Tumorerkrankungen des Gastrointestinaltraktes zählen zu den häufigsten Ursachen des Todes an einem Krebsleiden beim Menschen.

Mit Tumormarkern könnten bspw. Primärtumoren zu einem frühen Zeitpunkt darauf untersucht werden, ob sie metastasierend werden oder nicht, was ein frühzeitiges Eingreifen und im günstigsten Fall eine Prävention der Metastasenbildung ermöglichen kann.

Zhang et al. ("Proteomic Study Reveals That Proteins Involved in Metabolic and Detoxification Pathways Are Highly Expressed in HER-2/neu-positive Breast Cancer", Mol.Cell. Proteom. 4.11:1686-1696, (2005)) beschreiben eine erhöhte Proteinexpression von PGK1 in Proben aus Her-2/neu-positiven Brusttumoren, und dass diese Tumoren eine erhöhte Wahrscheinlichkeit für eine Metastasierung haben.

Wang et al. ("A glycolytic mechanism regulating an angiogenic switch in prostate cancer", Can.Res. 67:149-159 (2007)) beschreiben, dass die Expression von PGK1, CXCR4, CXCL12 und beta-Catenin gekoppelt sind und bei einem Tumorwachstum eine Rolle spielen.

De Lange et al. ("Phase II trial of cisplatin and gemcitabine in patients with advanced gastric cancer", Ann.Oncol. 15:484-488 (2004)), beschreiben eine Therapie mit einer Kombination aus Gemcitabine und Cisplatin bei Peritonealkarzinosen.

Krishnan et al. ("Phsophorylation of pyrimidine deoxynucleoside analog diphosphates: selective phosphorylation of L-nucleoside analog diphosphates by 3-phosphoglycerate kinase", J.Biol.Chem. 277:5453-5459 (2002)) untersuchten die Rolle von u.a. 3-Phosphoglycerat-Kinase in der Phosphorylierung von L-Nucelosid-Diphosphaten.

Rose et al. ("Gemcitabine reverses cisplatin resistance: Demonstration of activity in platinum- and multidrug-resistant ovarian and peritoneal carcinoma", Gyn.Oncol. 88:17-12 (2003)) beschreiben den kombinierten Einsatz von Gemcitabine und Cisplatin und zeigten, dass mit dieser Kombination Patienten erfolgreich behandelt werden konnten, die zuvor gegenüber Gemcitabin resistent waren.

Die WO 2007/065010 A2 offenbart die Behandlung von Magenkarzinomen mittels eines HIF-Inhibitors, der auch die Expression von PGK1 inhibieren kann.

Sun et al. ("Detection of type IV collagenase activity in malignant ascites", J.Gastr. 9:2592-2595 (2003)) beschreiben eine erhöhte Expression der Typ IV Collagenase in malignen Asziten bei Peritonealkarzinosen.

Kress et al. ("Expression of hypoxia-inducible genes in tumor cells", J.Cancer Res.Clin.Oncol. 124:315-320 (1998)) beschreiben eine erhöhte PGK1-Expression in colorectalen Krebsproben.

Daher ist Aufgabe der vorliegenden Erfindung, neue Marker für Peritonealkarzinomen sowie neue Angriffspunkte für deren Therapie bereitzustellen. Diese Aufgabe wird erfindungsgemäß gelöst durch die Verwendung von Phosphoglycerat-Kinase 1 (PGK1) wie in den Ansprüchen 1 und 6 definiert. Dabei kann PGK1 entweder alleine, oder aber auch, in einer Ausführungsform der Erfindung, in Kombination mit beta-Catenin, CXCR4 und/oder CXCL12 als Marker verwendet werden. Darüber hinaus wird die Aufgabe durch eine pharmazeutische Zusammensetzung zur Behandlung von Peritonealkarzinosen, wie in den Ansprüchen 12 und 15 gelöst, mit der die Überexpression von PGK1 alleine, oder aber in Kombination mit beta-Catenin, CXCR4 und/oder CXCL12, inhibiert wird.

Die der Erfindung zugrundeliegende Aufgabe wird auf diese Weise vollkommen gelöst. Durch eingehende vergleichende Analysen zwischen bösartig entartetem Gewebe und nicht entartetem Gewebe konnten Gene und Proteine identifiziert werden, die sich bei den Gewebearten in ihrer Häufigkeit bzw. Konzentration signifikant unterscheiden. Die quantitative Expression eines bestimmten Gens oder Proteins im Vergleich mit Kontrollen, insbesondere der in der vorliegenden Anmeldung beanspruchten Gene und Proteine, bildet damit ein wichtiges Indiz für das Vorliegen von Tumorgeweben, insbesondere metastasierenden Primärtumoren, wodurch diese Gene und/oder Proteine als diagnostische Marker verwendet werden können.

Der Erfinder hat in eigenen Versuchen erkannt, dass die genannten Gene/Proteine insbesondere bei metastasierenden Primärtumoren als Marker dahingehend für eine Diagnose/Vorhersage geeignet sind, ob diese Tumore Metastasen bilden werden oder nicht.

Dabei versteht sich, dass PGK1 entweder alleine als Marker für die genannten Tumorerkrankungen eingesetzt werden kann, oder aber in Kombination mit einem oder mehreren der Marker beta-Catenin, CXCR4 oder CXCL12.

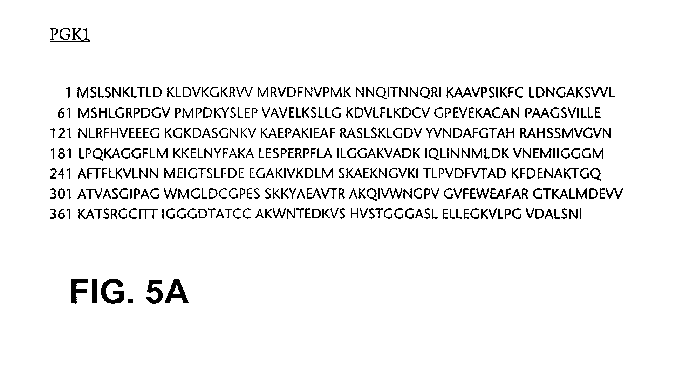

Phosphoglycerat-Kinase 1 - im Folgenden auch lediglich als "PGK1" bezeichnet - ist ein Enzym, das die reversible Umwandlung von 3-Phosphoglycerat zu 1,3-Diphosphoglycerat und ADP katalysiert. Die physiologische Bedeutung des Enzyms liegt vor allem in der Umkehrreaktion, durch welche die Energie des bei der Glycolyse anfallenden 1,3-Diphosphoglycerats in Form von ATP für Energie-verbrauchende Stoffwechselreaktionen bereitgestellt wird. Die Expression von PGK1 wird durch den durch Hypoxie induzierbaren Faktor 1α (HIF-1α) reguliert. Die Proteinsequenz von PGK1 ist in Fig. 5A wiedergegeben (oben) und in dem beiliegenden Sequenzprotokoll mit der SEQ-ID Nr. bezeichnet, die mRNA von PGK1 (siehe Fig. 5A, unten) mit der SEQ-ID Nr. 2.

PGK1 spielt nicht nur als glycolytisches Enzym eine Rolle, sondern auch als Suppressor von proangiogenen Faktoren, wie bspw. VEGF ("vascular endothelial cell growth factor") und Interleukin IL-8. Abgesehen davon wurde aufgrund einer neueren Studie angenommen, dass PGK1 bei der Entstehung maligner Prostata-Tumoren beteiligt ist (siehe Wang et al., "A glycolytic mechanism regulating an angiogenic switch in prostate cancer", Cancer Research, 67: 149-159, 2007). Ferner konnten verschiedene Arbeitsgruppen eine Überexprimierung von PGK1 bei Brust- und Pankreaskarzinomen beobachten, sowie bei Gebärmutterkrebs, jedoch bis heute nicht bei metastasierenden Primärtumoren des Gastrointestinaltraktes.

Beta-Catenin, ein 92 kD Protein, spielt - neben seiner Bedeutung für den Zusammenhalt von Gewebeverbänden und die Kommunikation von Zelle zu Zelle - auch eine wichtige Rolle in der Signalübertragung innerhalb der Zelle, bei der Zellvermehrung, der Wachstumsregulation sowie der embryonalen Entwicklung. Beta-Catenin bindet in einer gesunden Zelle an mehrere Proteine und bildet mit diesen Transkriptionsfaktoren, die eine Stimulierung der Zellvermehrung und/oder eine Unterdrückung des Zelltods bewirken. Beta-Catenin ist außerdem an der Entstehung verschiedener Krebsformen beteiligt, wie bspw. bei der familiären adenomatösen Prolypose, bei der es aufgrund einer Funktionsunfähigkeit des Tumorsuppressors APC, der normalerweise überschüssiges beta-Catenin abfängt, zu einer verstärkten Zellvermehrung kommt. Bei Darmkrebs und einigen Hautkrebszelllinien findet man Mutationen im beta-Catenin selbst, wodurch die Inaktivierung des beta-Catenins durch Phosphorylierung gestört wird.

Bis heute wurde jedoch keine Verbindung zwischen der Expression von PGK1 und beta-Catenin bei Magenkrebs oder bei der peritonealen Dissemination des Magenkrebses beschrieben.

Chemokine sind sekretierte Zytokine, sie sich aufgrund struktureller Unterschiede in vier Gruppen einteilen lassen, darunter die CXC-Gruppe. Chemokinrezeptoren weisen eine bestimmte Struktur auf: es handelt sich um G-Protein-gekoppelte Rezeptoren, die mit 7-Transmembran-alpha-Helices die Zellmembran durchspannen. Das bekannte menschliche Chemokinsystem enthält über 50 Liganden und 20 G-Protein-gekoppelte Rezeptoren, die die Migration und Aktivierung von Leukozyten steuern sowie die Angiogenese und das Tumorwachstum beeinflussen. Im Stand der Technik wird auch angenommen, dass Tumorzellen selber Chemokine sekretieren können. Disseminierte Tumorzellen, die Mitglieder der CXCR-Familie exprimieren, und in die Zirkulation eindringen, werden daher durch ihren korrespondierenden Liganden eingefangen. Daher gewinnen diese Zellen die Fähigkeit, in bestimmte Organe einzudringen.

Der Chemokinrezeptor CXCR4, der den Rezeptor für das Chemokin CXCL12 darstellt, wird auf verschiedenen Tumorzellen exprimiert, z.B. auch auf Zellen von Patienten mit Chronisch Lymphatischer Leukämie (CLL) sowie auf Primärtumoren und Zelllinien des Kleinzelligen Bronchialkarzinoms (SCLC). Ferner ist inzwischen bekannt, dass Chemokine auch das Wachstum und die Metastasenbildung mehrerer maligner Tumoren fördern können. Daher kann die Expression von Chemokinrezeptoren, insbesondere CXCR4, durch Tumorzellen ein wichtiger Faktor bei der organspezifischen Metastasenbildung sein. So wurde bspw. bei Brustkrebs und bei Prostatakrebs der Wechselwirkung zwischen CXCR4 und CXCL12 eine Rolle bei der Metastasenbildung zugeschrieben. Ferner untersuchten Yasumoto et al., "Role of the CXCL12/CXCR4 Axis in Peritoneal Carcinomatosis of Gastric Cancer", Cancer Res. 66: 2181-2187 (2006) die Rolle der CXCR4/CXCL12 Wechselwirkung bei Peritonealkarzinosen des Magenkrebs. Dass darüber hinaus bei Peritonealkarzinosen des Magenkrebs ein Zusammenhang zwischen der Expression von PGK1 und der Expression CXCR4/CXCL12 besteht, ist hingegen neu und bisher im Stand der Technik nicht beschrieben.

PGK1 stellt daher entweder alleine, oder aber in Kombination mit beta-Catenin, CXCR4, CXCL12, einen geeigneten Marker/eine geeignete Markerkombination zur Diagnose/Vorhersage von Peritonealkarzinosen dar, wie der Erfinder in eigenen Versuchen zeigen konnte.

In einer Ausführungsform der erfindungsgemäßen Verwendung ist bevorzugt, wenn eine Hochregulierung des PGK1-Gens und/oder PGK-1-Proteins untersucht wird, oder alleine oder in Kombination mit der Hochregulierung von beta-Catenin, CXCR4 und/oder CXCL12.

Unter "Hochregulierung" oder "erhöhte Expression" wird vorliegend dabei jede Genoder Proteinexpression von PGK1, CXCR4, CXCL12 und beta-Catenin verstanden, die im Vergleich zur normalen Expression der zu untersuchenden Gene in gesunden Proben erhöht bzw. verstärkt ist. Der Vergleich der unterschiedlichen Expression kann dabei mit Kontrollen oder Standards stattfinden. Eine "hoch regulierte" oder "erhöhte" Genexpression bedeutet, dass das betreffende Gen stärker als üblich für dieses Gen transkribiert - und ggf. auch in das entsprechende Protein translatiert-wird, was auf verschiedene Faktoren zurückzuführen sein kann. Daher versteht sich, dass die vorliegende Erfindung sowohl die Untersuchung der für PGK1, CXCR4, CXCL12 und beta-Catenin codierenden DNA-Sequenzen umfasst, als auch die Untersuchung der Häufigkeit der Proteine selber, oder der diesen zugrunde liegenden RNA, umfasst.

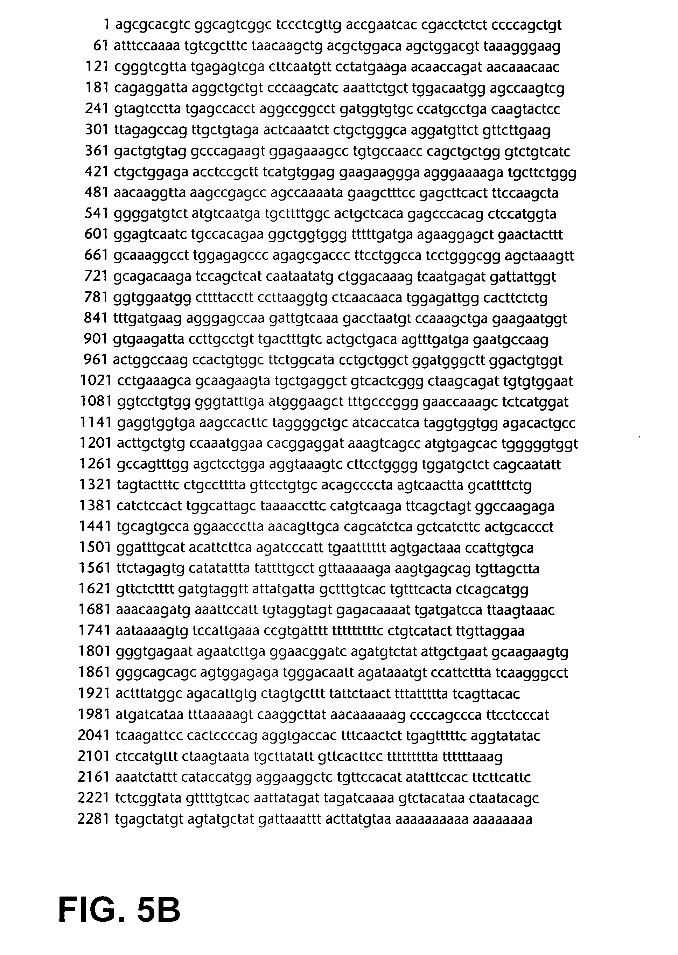

Die Proteinsequenz des Markers PGK1 findet sich in Fig. 5A, die Sequenz der mRNA in Fig. 5B dieser Anmeldung. Diese Sequenzen, sowie die für das PGK1-codierende Gensequenz sind in öffentlich zugänglichen Datenbanken aufgeführt. So wird bspw. die mRNA von PGK1 in der Datenbank ("Genbank") des National Center for Biotechnology Information ("NCBI"; http://www.ncbi.nlm.nih.gov/) unter der Datenbank-Nr. NM_00291 geführt, die mRNA von CXCR4 unter NM_003467, die mRNA von CXCL12 unter NM_001033886 und die mRNA von beta-Catenin unter NM_001904. Über diese mRNA-Datenbanknummern lassen sich ebenfalls die den Markern entsprechenden Gen- und Proteinsequenzen identifizieren.

Vorliegend wird durch die vorliegende Anmeldung hindurch der Begriff "Marker" für sowohl das betreffende Protein/Genprodukt, als auch das für dieses codierende Gen verwendet, so dass der Begriff "Marker" Protein, Genprodukt, und/oder Gen von PGK1, CXCR4, CXCL12, und beta-Catenin bedeuten kann.

Diese Untersuchungen können - hinsichtlich der Genexpression - bspw. Polymerasekettenreaktion (PCR) sein, ferner Genchip-/Micorarraysysteme, RNAse Protection Assays, u.a., und insbesondere jedes molekularbiologische Verfahren, mit welchen bestimmte DNA- oder RNA-Sequenzen amplifiziert werden können. Dadurch kann ein quantitativer und ein qualitativer Nachweis der zu untersuchenden Genprodukte auf DNA- oder RNA-Ebene erhalten werden. Daneben kann der Nachweis aber auch über Hybridisierungsversuche durch herkömmliche Northern- oder Southern-Blots erfolgen, mit welchen ebenfalls quantitative/qualitative Aussagen über die Abundanz von Proteinen auf DNA- oder RNA-Ebene getroffen werden können.

Daneben können die Marker auf Protein-Ebene durch herkömmliche Western-Blots, Protein-Chips, mit Hilfe von Antikörpern und/oder Immuno-Assays, wie bspw. ELISA (enzyme-linked immunosorbent assay), immunohistochemische Verfahren, etc., qualitativ und quantitativ bestimmt werden.

Die Genexpression kann durch die Verwendung der Sequenzen, oder Fragmente davon, aus dem beiliegenden Sequenzprotokoll, nämlich die SEQ ID Nr. 1 bis 6, 15 und 16, zur Identifizierung der diagnostischen Marker zur Vorhersage oder Diagnose von Peritonealkarzinosen nachgewiesen werden.

Die beanspruchten Sequenzen stellen Primer dar, mit denen die jeweiligen Genprodukte PGK1, CXCR4, CXCL12 und beta-Catenin in der quantitativen Real Time PCR-Analyse identifiziert wurden. Es versteht sich, dass im Rahmen der vorliegenden Erfindung auch jeder andere Primer für die erfindungsgemäße Verwendung geeignet ist, mit Hilfe dessen sich die Genprodukte PGK1, CXCR4, CXCL12 und beta-Catenin identifizieren lassen, so dass auch die beanspruchten Primer leicht modifiziert sein können, bspw. hinsichtlich ihrer Länge oder übliche Veränderungen in der Sequenz, aber gleichzeitig ihre Funktion zum Nachweis der Genprodukte beibehalten.

Ferner betrifft die Erfindung ein Verfahren zur Vorhersage oder Diagnose von Peritonealkarzinosen, wobei das Verfahren den *in-vitro*-Nachweis einer erhöhten Genoder Proteinexpression von Phosphoglycerat-Kinase 1 (PGK1) alleine oder in Kombination mit einer erhöhten Gen- oder Proteinexpression von CXCR4, CXCL12 und/oder beta-Catenin in einer biologischen Probe umfasst.

Mit dem erfindungsgemäßen Verfahren kann eine zuverlässige Aussage über die Neigung von Primärtumoren, Metastasen zu bilden, getroffen werden, da mit den erfindungsgemäßen Markern das entsprechende Werkzeug hierfür bereitgestellt wird.

Insbesondere ist bevorzugt, wenn das erfindungsgemäße Verfahren die folgenden Schritte umfasst:
a) Bereitstellen einer biologischen Probe eines zu untersuchenden Patienten, der an einem Tumor erkrankt ist,
b) Nachweis einer erhöhten Gen- und/oder Proteinexpression von zumindest einem der folgenden Gene oder Proteine, Phosphoglycerat-Kinase 1 (PGK1), CXCR4, CXCL12 oder beta-Catenin in den biologischen Proben im Vergleich zu Kontrollen und/oder Standards.

Dabei kann die biologische Probe eine flüssige oder feste Körperprobe eines Patienten sein, insbesondere eine Probe von Blut, Serum, Plasma, Urin, Gewebe, Knochen, Knorpel, Organe, etc. Die Gewebeprobe kann dabei Probe eines Primärtumors sein, und die Kontrolle entweder die Probe eines nicht-metastasierenden Tumorgewebes oder Standards. Die Proben können dabei gemäß den Fachleuten gängigen Verfahren entnommen und ggf. aufbereitet werden.

Bei dem erfindungsgemäßen Verfahren kann, wie bereits weiter oben aufgeführt, der Nachweis bspw. über die Hilfsmittel Polyacrylamid-Gelelektrophorese, Immunohistochemie, ELISA, (enzyme-linked immunosorbent assay), Gen-/Protein-Micorarrays, PCR, etc., durchgeführt werden. Die genannten Verfahren sind im Stand der Technik, insbesondere im Gebiet der medizinischen Forschung als Standardverfahren bekannt. Anleitungen zur Durchführung dieser Verfahren sowie weiterer, die hier vorliegend nicht aufgeführt sind, finden sich bspw. in Sambrook, Fritsch, Maniatis, "Molecular cloning, a laboratory manual", auf das hiermit explizit verwiesen wird.

Offenbart wird ein Diagnosekit verwendet werden, umfassend zumindest eine Substanz zum Nachweis der Aktivität und/oder Expression von Phosphoglycerat-Kinase 1 (PGK1) alleine oder in Kombination mit CXCR4, CXCL12 und/oder beta-Catenin zur Diagnose oder Vorhersage von metastasierenden Primärtumoren oder Peritonealkarzinosen.

Mit einem solchen Diagnosekit kann die Abundanz der Marker in biologischen Proben bestimmt werden, und zwar durch einen Vergleich der Gen-/Proteinexpression der zu untersuchenden, den oder die Marker enthaltenden Probe mit Kontrollen oder Standards, insbesondere hinsichtlich einer erhöhten Expression des/der Marker. Das Diagnosekit ist daher insbesondere zur Früherkennung von metastasierenden Primärtumoren geeignet, was wiederum eine rasche und gezielte Behandlung ermöglicht. Mit dem Diagnosekit kann somit bspw. auch zwischen nicht-metastasierenden Magentumoren und metastasierenden Magentumoren unterschieden werden. Das Diagnosekit enthält dabei bspw. einen oder mehrere Antikörper oder Oligonucleotide, die mit dem erfindungsgemäßen Marker/Markerkombinationen auf Protein- oder Gen-Ebene reagieren.

Offenbart wird ferner die Verwendung von Phosphoglycerat-Kinase 1 alleine oder in Kombination mit CXCR4, CXCL12 oder beta-Catenin, als Targets für die therapeutische oder präventive Behandlung von Peritonealkarzinosen.

Die Erfindung betrifft ferner die Verwendung wie in Anspruch 12 definiert, von einem Wirkstoff, der die Aktivität und/oder Expression von Phosphoglycerat-Kinase 1 (PGK1) hemmt, entweder alleine oder in Kombination mit einem Wirkstoff, der die Aktivität und/oder Expression von CXCR4, CXCL12 oder beta-Catenin hemmt, und/oder in Kombination mit zumindest einem Chemo- und/oder Radiotherapeutikum, zur Herstellung eines Arzneimittels für die Behandlung und/oder Prävention von Peritonealkarzinosen.

Die Marker sind neben ihrer Eignung als diagnostische Marker auch als Targets, also Ziele, für eine Behandlung mit einem Wirkstoff geeignet, der die Aktivität und/oder Expression der Marker hemmt. Wie weiter oben beschrieben, ist die Abundanz der genannten Marker in metastasierenden Primärtumoren gegenüber nicht-metastasierenden Primärtumoren erhöht. Die Beeinflussung der Aktivität oder Expression dieser Marker bis hin zur vergleichsweise normalen Aktivität/Expression stellt daher einen Behandlungsansatz des als solchen identifizierten metastasierenden Primärtumors und/oder Ansatz zur Vorbeugung der Metastasierung des Primärtumors dar.

Der Wirkstoff, mit dem die Aktivität und/oder die Expression der genannten Marker gehemmt werden kann, kann ein spezifischer Antikörper sein, bei welchem es sich um einen blockierenden Antikörper oder um einen radioaktiv markierten Antikörper handelt, mit welchem man eine sog. Radio-Immuntherapie durchführen kann.

Bei den genannten Proteinen bzw. Genen wurde in eigenen Versuchen gezeigt, dass sie in charakteristischer Weise im Tumorgewebe im Vergleich mit Kontrollen heraufreguliert waren, also eine erhöhte Genexpression besaßen. Daher können entsprechende Wirkstoffe eingesetzt werden, die eine Herunterregulierung der Genexpression bewirken, um so eine Expression bzw. Aktivität der genannten Gene/Proteine von gesunden Geweben zu erreichen. Andererseits können auch Wirkstoffe eingesetzt werden, die bspw. wie blockierende/inhibierende Antikörper auf die Marker wirken, und dadurch deren Aktivität hemmen. Die einzusetzenden Antikörper sind dabei bspw. monoklonale Antikörper und insbesondere rekombinante, humanisierte Antikörper oder chimäre Antikörper. Herkömmliche monoklonale Antikörper enthalten neben der die Spezifität gegen humane Antigene vermittelnden variablen Region auch Proteinbestandteile der Maus; daher werden die konstanten Abschnitten der Maus-Antikörper durch molekularbiologische Verfahren entfernt und durch baugleiche konstante Teile menschlicher Antikörper ersetzt.

Die eingesetzten Wirkstoffe können bspw. Peptide, Proteine, sog. Small Molecular Compounds oder Polynucleotide sein, und synthetisch oder natürliche Wirkstoffe sein. Die Wirkstoffe können entweder direkt an den jeweiligen Proteinen oder aber bereits in der Genexpression angreifen. Erfindungsgemäß kommen Short Molecules in Frage, nämlich si-RNA ("small interfering RNAs"), und entsprechende Antisense-Oligonucleotide, wie antisense-RNA oder -DNA, die mit den entsprechenden Genen bzw. Genprodukten der Marker interferieren und diese hemmen können.

Die Wirkstoffe können dabei auch bspw. eine blockierende Wirkung besitzen, so dass Wirkstoffe eingesetzt werden können, die bspw. Rezeptorstellen (wieCXCR4) besetzen, wodurch die Wechselwirkung des Rezeptors mit seinem (seinen) üblichen Liganden unterbrochen ist.

Das Therapeutikum, das zusammen mit einem gegen PGK1 gerichteten Wirkstoff eingesetzt werden kann, kann ein gegenwärtig zur Krebsbehandlung üblicherweise verwendetes Chemo- und/oder Radiotherapeutikum sein, wie bspw. radioaktivmarkierte, insbesondere mit ¹²⁵I markierte, Wirkstoffe, wie bspw. Antikörper sein, sowie natürlich vorkommende (bspw. alpha-Interfreon, Interleukin-12) oder synthetisch hergestellte Substanzen sein. Als Chemotherapeutikum können antineoplastisch wirkende Cytostatika eingesetzt werden. So sind gegenwärtig bspw. Quercetin, Tamoxifen oder Staurosporin, die effektive Hemmstoffe der Proteinkinase C sind, als Chemotherapeutika bei der Krebsbehandlung bekannt. Ferner ist die Stoffgruppe der Di-Triazene entwickelt worden, derer Wirkung auf einer Interaktion mit der DNA beruht.

Schließlich betrifft die vorliegende Erfindung eine pharmazeutische Zusammensetzung umfassend einen Wirkstoff, wie in den Ansprüchen definiert, der die Aktivität und/oder Expression von PGK1, entweder alleine, oder in Kombination mit einem Wirkstoff, der die Aktivität/Expression von CXCR4, CXCL12 oder beta-Catenin hemmt, sowie ggf. zumindest einen pharmazeutisch akzeptierbaren Träger.

Die erfindungsgemäße Zusammensetzung kann bspw. systemisch, wie bspw. intravenös, oral oder subkutan, oder aber lokal verabreicht werden, und die Wirkstoffe dabei entweder zusammen in einer pharmazeutischen Zusammensetzung oder aber separat in mehreren Zusammensetzungen mit einer aufeinander folgenden Applikation verabreicht werden.

Die Zusammensetzung kann dabei weitere Inhaltsstoffe aufweisen, die üblicherweise in pharmazeutischen Zusammensetzungen zu finden sind, und von der Verabreichungsform, dem Patienten, etc, abhängen können. Eine Reihe von geeigneten Substanzen findet sich in A. Kibbe, Handbook of pharmaceutical excipients, 3. Ed., 2000, American Pharmaceutical Association and Pharmaceutical press.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Vorteile ergeben sich aus den nachstehenden Beispielen sowie den Figuren. Es zeigen:
- Fig. 1:: die Auflistung von Primer-Sequenzen (menschlich) für die quantitative Real-Time-PCR-Analyse in Tabelle 1;
- Fig. 2:: eine Übersicht über differentiell regulierte Gene aus den Mikroarray-Versuchen, die mit der quantitativen Real Time PCR (qRT-PCR) gepoolter RNA-Proben der gleichen Patienten evaluiert wurden, wie sie für die Mikroarray-Versuche eingesetzt wurden. Die angegebenen Veränderungen in der Häufigkeit beziehen sich auf Gene, die für diffuse Primärtumore von Magenkrebs mit Peritonealkarzinosen spezifisch sind (Tabelle 2);
- Fig. 3:: die Tabelle 3, in der ein Versuch zusammengefasst ist, in welchem ausgewählte Gene mittels quantitativer Real Time PCR (qRT-PCR) einzelner Proben evaluiert wurden. Die angegebenen Änderungen in der Häufigkeit beziehen sich auf differentiell regulierte Gene, die spezifisch für diffuse Primärtumore von Magenkrebs mit Peritonealkarzinosen sind (Tabelle 3);

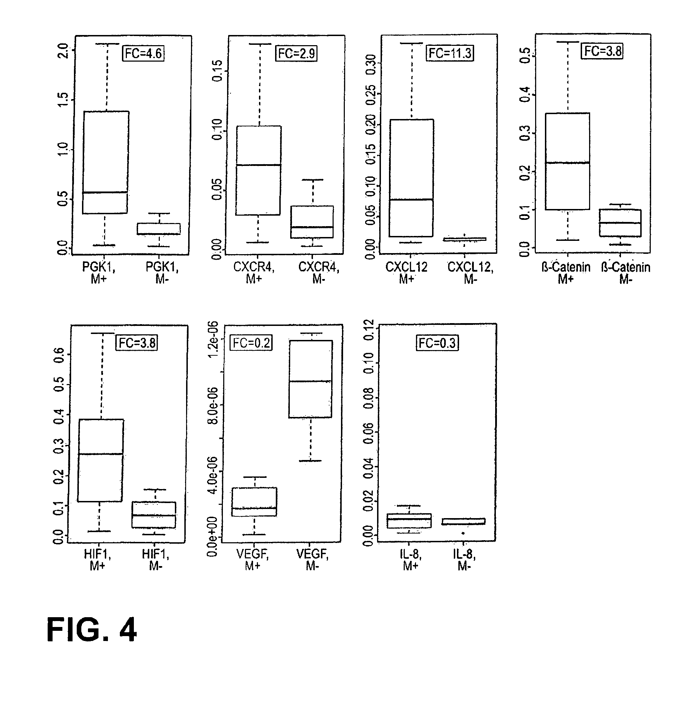

- Fig. 4:: Boxplots quantitativer Real-Time-PCR-Messungen einzelner Patienten RNA-Proben. In den Boxplots ist jeweils das Minimum, der Viertelwert, der Mittelwert, der Drittelwert und das Maximum der zugrundeliegenden Verteilung der qRT-PCR-Werte dargestellt. Für jedes Gen wurden zwei Boxplots aufgezeichnet, wobei das linke sich jeweils auf Magentumorproben mit Peritonealkarzinosen (M+) bezieht, und das rechte jeweils auf Proben ohne Peritonealkarzinosen (M-); darüber hinaus ist der Mittelwert der Häufigkeitsänderung der Peritonealkarzinose-positiven Proben gegenüber den Negativproben für jedes Gen gezeigt;
- Fig. 5:: Sequenz des PGK1-Proteins (A) und der PGK1-mRNA (B);

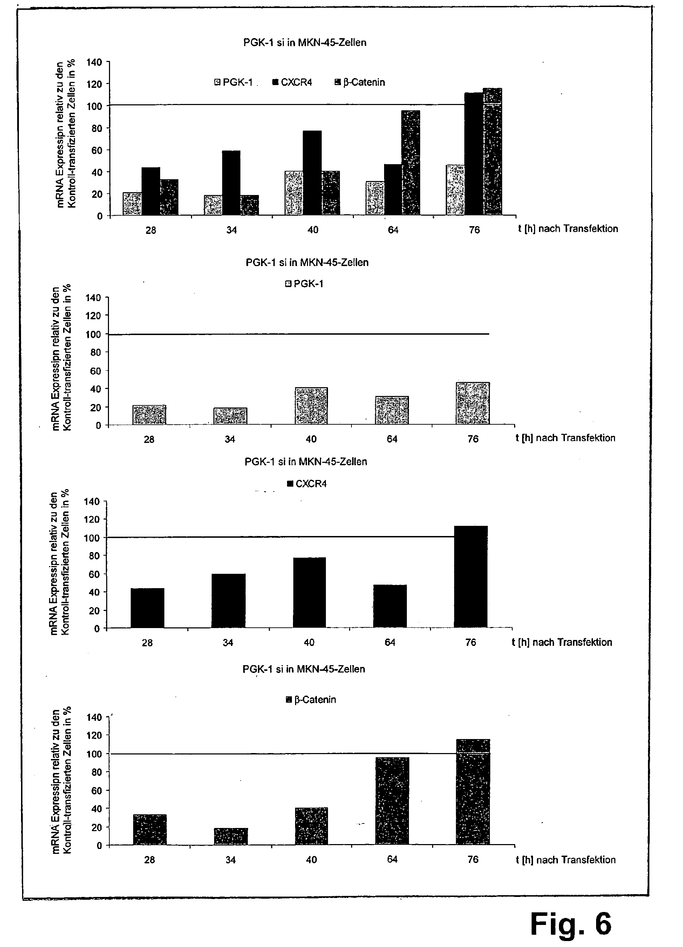

- Fig. 6:: Ergebnisse von Genedownsilencing-Versuchen mittels si-RNA auf der mRNA-Ebene: PGK1 hat einen Einfluss auf die Regulation der Signalmoleküle CXCR4, CXCL12 und beta-Catenin und VEGF und *vice versa.*
- Fig. 7:: Ergebnisse von Genedownsilencing-Versuchen mittels si-RNA auf der Proteinebene; gezeigt sind Western-Blot-Ergebnisse (Fig. 7A) und FACS-Auswertungen (Fig. 7B).

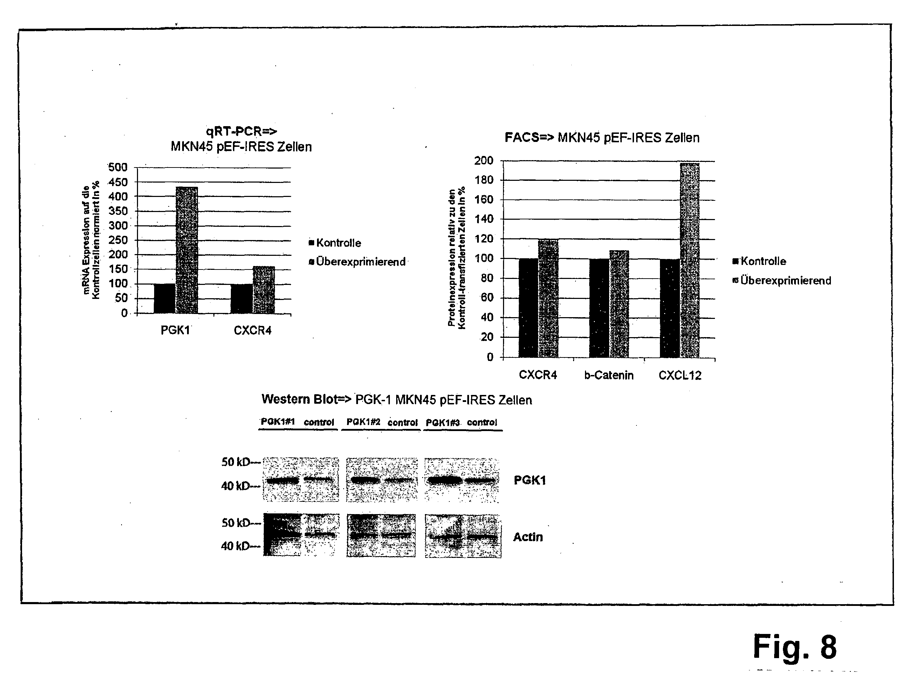

- Fig. 8:: Ergebnisse der Überexpression von PGK1 (Transfektion von PGK1). Links oben: Die Ergebnisse auf Genexpressionsebene mittels Real-Time-PCR von PGK1 und CXCR4. Man sieht, dass PGK1 mRNA sowie CXCR4 mRNA im Vergleich zur Kontrolle ohne Transfektion hochreguliert sind. Rechts oben: Die Ergebnisse von CXCR4, b-Catenin und CXCL12 auf Proteineben mittels FACS. Man sieht, dass diese Gene in den Zellen mit PGK1-Transfektion auch wieder im Vergleich zu den Kontrollen hochreguliert sind. Unten: Beweis, dass PGK1 auch auf Proteinebene in den transfizierten Zellen im Vergleich zu den Kontrollen vermehrt vorhanden ist.
- Fig. 9:: Untersuchungen zur Invasivität transfizierter und nicht-transfizierter Zellen Links: 100-fache Vergrößerung: Magenkarzinomzellen MKN45 ohne PGK1-Überexpression. Rechts: 100-fache Vergrößerung: Magenkarzinomzellen MKN45 mit PGK1-Überexpression.
- Fig. 10:: die Tabelle 4, in der die Sequenzen der für die Genedownsilencing-Versuche verwendete siRNAs gezeigt sind.

### Ausführungsbeispiel

### 1. Material und Methoden

### a: Patienten, Gewebeproben und RNA-Extraktion

Gewebeproben von 13 (unbehandelten, ohne jegliche Neoadjuvans-Therapie) Patienten mit histologisch nachgewiesenem diffusen Magenkrebs, die einer Laparatomie unterzogen wurden, wurden untersucht. Bei 8 von 13 Patienten wurde eine Peritonealkarzinose histologisch nachgewiesen (5 weibliche, 3 männliche Patienten; Durchschnittsalter 58 Jahre, Altersbereich 27-78). Bei 5 von 13 Patienten konnte keine Peritonealkarzinose nachgewiesen werden (4 weibliche, 1 männlicher Patient; Durchschnittsalter 70 Jahre, Altersbereich 60-76). Alle Tumorproben wurden durch die Abteilung der Allgemeinen, Viszeral- und Transplantationschirurgie, Universität Tübingen, Deutschland, gesammelt. Die Proben wurden in flüssigem Stickstoff schockgefroren und bei -80 °C bis zur Verwendung gelagert. Jede Tumorprobe wurde kryogeschnitten, mit Hämatoxylin und Eosin gefärbt, durch zwei erfahrene Pathologen klassifiziert und durch einen erfahrenen chirurgischen Pathologen re-evaluiert. Die RNA wurde unter Verwendung des NucleoSpin RNA II Kits (Macherey-Nagel, Düren, Deutschland) extrahiert. Die RNA-Qualität und -Quantität wurde unter Verwendung des Agilent 2100 Bioanalyzer (Agilent Technologies, Palo Alto, USA) und des NanoDrop ND-1000 Spektrophometers (NanoDrop Technologies, Wilmington, Delaware, USA) bestätigt.

### b: Microarray-Datengenerierung

Microarray-Daten wurden unter Verwendung von Oligonucleotid-Microarrays (65mer) gesammelt, welche im Max-Planck-Institut Tübingen, Deutschland, hergestellt wurden. Die Array enthielten jeweils Oligonucleotide für ungefähr 900 Transkripte, wobei jedes Oligonucleotid zweimal auf dem Array vorlag. In dem vorliegenden Versuch wurden Primärtumore von Patientenproben mit Magenkrebs mit und ohne Entwicklung von Peritonealkarzinosen verglichen (n = 6; 3 Patienten, die Peritonealkarzinose entwickelten, im Vergleich mit 3 Patienten, die keine Peritonealkarzinose entwickelten). Um die Variabilität aufgrund der beiden Farbstoffe zu minimieren, wurden 9 Microarrays aus 6 Proben durchgeführt. Weitere Details des Arrays sind vom "National Center for Biotechnology Information's Gene Expression Omnibus (http://www.ncbi.nlm.nih.gov/geo/) unter der Zugangsnummer GPL5676 erhältlich. Die Amplifizierung der RNA-Probe wurde mit Ambion's Amino Allyl MessageAMP™ II RNA Amplification Kit (Ambion Inc., Austin, USA) durchgeführt. Farbstoffkopplungsreaktionen wurden unter Verwendung des Amersham CyDye Postlabelling Reactive Dye Pack (GE Healthcare, Buckinghamshire, Großbritannien) durchgeführt. Nach einer RNA-Fragmentierung durch Ambion-Fragmentierung-Reagenzien wurde eine Hybridisierung bei 48 °C für 14 Stunden durchgeführt.

### c: Quantitative Real Time PCR (qRT-PCR)

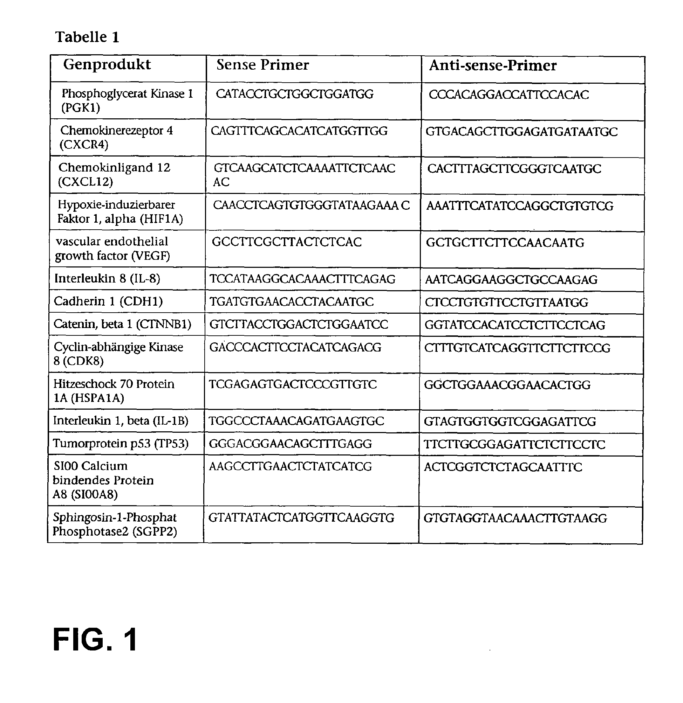

Die Proben-RNA wurde mit dem Transcriptor First Strand cDNA Synthese-Kit (Roche, Mannheim, Deutschland) transkribiert. Ausgewählte cDNAs wurden mittels Real Time PCR auf einem LightCycler®-Instrument (Roche, Mannheim, Deutschland) quantifiziert. Für die PCR-unterstützte Amplifizierung wurde der SYBR Green Jump Start TAQ ReadyMix (Sigma, Taufkirchen, Deutschland) gemäß den Anleitungen des Herstellers verwendet. Die Spezifität der PCR-Bedingungen wurde durch Bestimmung der Schmelztemperatur und die Sequenzierung des Produktes bestätigt. Die Quantifizierung der Level der Genexpression wurde mittels eines Vergleichslaufs einer externen Standardkurve zusammen mit den Proben von Interesse durchgeführt. Die verwendeten Primer-Sequenzen sind in der Tabelle 1 der Fig. 1 gezeigt.

### d: Statistische Analyse

Die Sammlung der Rohdaten wurde unter Verwendung eines ImaGene v.5.0 durchgeführt. Weitere statistische und bioinformatische Analysen wurden unter Verwendung der R-Sprache (www.r-project.org) und des Limma-Pakets des Bioconductor-Projekts (http://www.bioconductor.org/) durchgeführt. Als erster Schritt der Signalextraktion wurde für jeden Kanal der Mittelwert der Pixelverteilung für das Vordergrundsignal verwendet, sowie der Mittelwert für den Hintergrund jedes Spots als Schätzwerte für die Rohsignalwerte. Alle Spots wurden ohne Berücksichtigung ihres Flag-Status berücksichtigt. Der Versuch wurde doppelt ausgeführt, mit 9 Arrays von 6 Proben. Die Daten wurden unter Verwendung der Loess-Normalisierung normalisiert, und zwar mit Expressionswerten, korrigiert hinsichtlich des Normexp-Hintergrunds, gefolgt von einer Farbstoff-Swap-Normalisierung und einer anteiligen Normalisierung bezüglich Zwischenraum-Arrays. Sowohl die Loess- und Anteils-Normalisierungsverfahren wurden, wie im Limma-Paket vorgesehen, eingesetzt. Als Ergebnis wurde das Log-Verhältnis M der Proben mit Peritonealkarzinose gegenüber Proben ohne Peritonealkarzinose für jeden Spot mit dem Rechner aufgezeichnet. Basierend auf den aufgezeichneten M-Werten, wurden die differentiell exprimierten Gene durch Anwendung des Welch-Ein-Proben-t-Tests wie in R. implementiert, detektiert.

### e: Genedownsilencing-Versuche

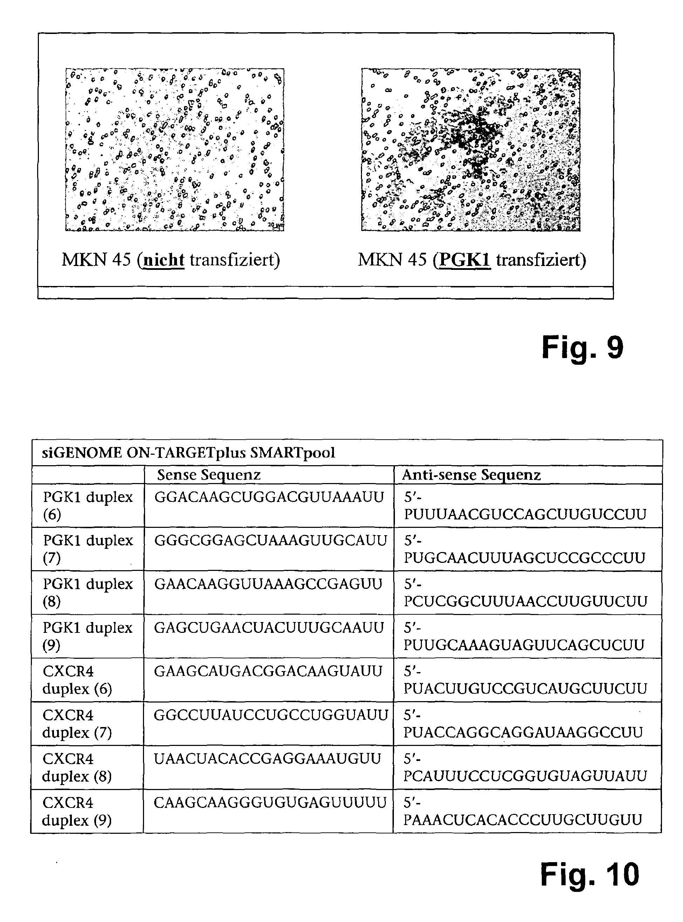

In Genedownsilencing-Versuchen sollte der Einfluss von PGK1 auf die Regulation von CXCR4/CXCL12/beta-Catenin/VEGF auf der mRNA- und der Proteinebene untersucht werden. Hierzu wurde si-RNA eingesetzt. Das posttranskriptionelle Genesiliencing erfolgte mit dem Lipid-basierten Transkriptionsreagenz Lipofectamine RNAiMAX (Invitrogen, Paisley, UK) nach Angaben des Herstellers in MKN-45 Zellen (Magenkrebszelllinie). Die verwendete siRNA (siGENOME ON-TARGETplus SMARTpool, Dharmacon, Chicago, USA) setzte sich aus vier verschiedenen Ziel-siRNAs für ein Gen zusammen, um eine höhere Silencing-Effizienz zu erzielen. Die Sequenzen der siRNA sind in der Tabelle 4 (Fig. 10) gezeigt. Als Kontrolle wurden gleichzeitig MKN-45 Zellen mit einer Kontroll-siRNA transfiziert, die keine Auswirkung auf die Expression von Genen hatte. Für den Verlauf der veränderten Genexpression wurden für die jeweiligen Versuchsansätze mehrere Zeitpunkte nach der Transfektion analysiert.

Die Genexpression auf mRNA-Ebene wurde mittels qRT-PCR untersucht. Dazu wurde mit dem RNeasy Mini Kit (Qiagen, Hilden, Deutschland) die Gesamt-RNA der transfizierten Zellen isoliert und wie in Unterpunkt c: beschrieben die qRT-PCR durchgeführt. Die verwendeten Primer sind in Tabelle 1 gezeigt.

Die Proteinexpression von PGK1 wurde mittels Western Blot analysiert. Für den Western Blot wurden die transfizierten Zellen mit RIPA Puffer (Pierce, Rockford, USA) nach den Herstellerangaben lysiert und die Gesamtproteine extrahiert. Für den Western Blot wurden 50 µg der Gesamtproteinmenge von jeder Probe auf ein 10% Polyacrylamid-Gel aufgetragen und mittels Gelelektrophorese ihrer Größe nach aufgetrennt. Geblottet wurden die Proteine auf eine PVDF-Membran (Millipore, Billerica, USA). Neben dem Primär-Antikörper für PGK1 (Abnova, Taipei, Taiwan) wurde auch b-Aktin (Affinity Bio Reagents, Golden, USA) als Kontrolle verwendet. Die Auswirkungen des Genesiliencing von PGK1 auf weitere Proteine wurden mittels Durchflusszytometrie (FACS) analysiert.

Die durchflusszytometrische Analyse der geernteten MKN 45 Zellen erfolgte mit jeweils 5x10⁵ Zellen mit mindestens 90% Vitalitätsquote pro Ansatz. Für intrazelluläre Färbungen verwendeten wir Intra Prep Permeabilisierungsreagenz (Beckman Coulter, Fullerton, USA) nach Herstellerangaben. Als Antikörper fanden Verwendung für die Determinierung der Proteinexpression von CXCR4 ein CXCR4 IgG2b Antikörper (R&D Minneapolis, USA) und ein Phycoerythrin gekoppelter Sekundärantikörper (BD, Franklin Lakes, USA). Für den Nachweis von CXCL12 wurde ein CXCL12 IgG1 Antikörper (R&D Mineapolis, USA) mit einem Phycoerythrin gekoppelten Sekundärantikörper (BD, Franklin Lakes, USA) verwendet. Der Proteinnachweis von beta-Catenin erfolgte mittels eines Alexa 488 markierten beta-Catenin Antikörpers (Cell Signaling, Danvers, USA). Für jeden Antikörper wurde eine entsprechende Isotypkontrolle verwendet. Die nach der Antikörperinkubation durch die oben genannten Fluorochrome markierten und gewaschenen MKN-45 Zellen wurden nun mittels eines Epics XL MCL Durchflusszytometers (Beckman Coulter, Fullerton, USA) gemessen. Vor der Messung wurde mittels Flow check beads Fluorosphären (Beckman Coulter, Fullerton, USA) der ordnungsgemäße Gerätezustand sichergestellt. Die Auswertung erfolgte unter Auswertung positiver Zellen bei der jeweils indizierten Anregungswellenlänge unter Normalisierung der Zellen durch einen Nullabgleich unspezifischer Fluoreszenz mit Hilfe der durch Isotypkontrollantikörper markierten MKN-45 Zellen.

### f: Überexpression

Zur Überexprimierung von PGK1 wurde das Gen in den Plasmid-Vektor pEF-IRES kloniert. Mit dem Transfektionsreagenz Tfx-50 (Promega, Madison, USA) wurde nach den Angaben des Herstellers das Plasmid in MKN-45 Zellen transfiziert. Nach 24 Stunden wurden die transfizierten Zellen in einem Selektionsmedium (RPMI 1640, 20% FCS und 5 µg Puromycin pro ml) kultiviert. Nach einer Woche endete die Zellselektion und die positiven Klone, welche den Plasmid-Vektor und das Resistenzgen stabil im Genom überexprimiert hatten, persistierten und wurden weiterkultiviert. Sobald ausreichend Zellen vorhanden waren, wurde mit dem RNeasy Mini Kit (Qiagen, Hilden, Deutschland) Gesamt-RNA isoliert, und entsprechend dem Vorgehen in Unterpunkt c: wurde mittels qRT-PCR die Expression auf mRNA-Ebene der stabil-transfizierten MKN-45 pEF-IRES Zellen mit kontroll-transfizierten MKN-45 Zellen verglichen. Die Genexpression auf Proteinebene wurde anhand von Western Blot und FACS-Analysen nachgewiesen. Die Durchführung erfolgte wie in Absatz e: beschrieben.

### Invasivität:

Die PGK1 überexprimiertern MKN-Zellen wurden auf ihre Invasivität im Vergleich zu den nicht transfizierten Zellen mittels einem gelatinebeladenen Migrationsassays in einer Boydenchamber analysiert.

### Ergebnisse

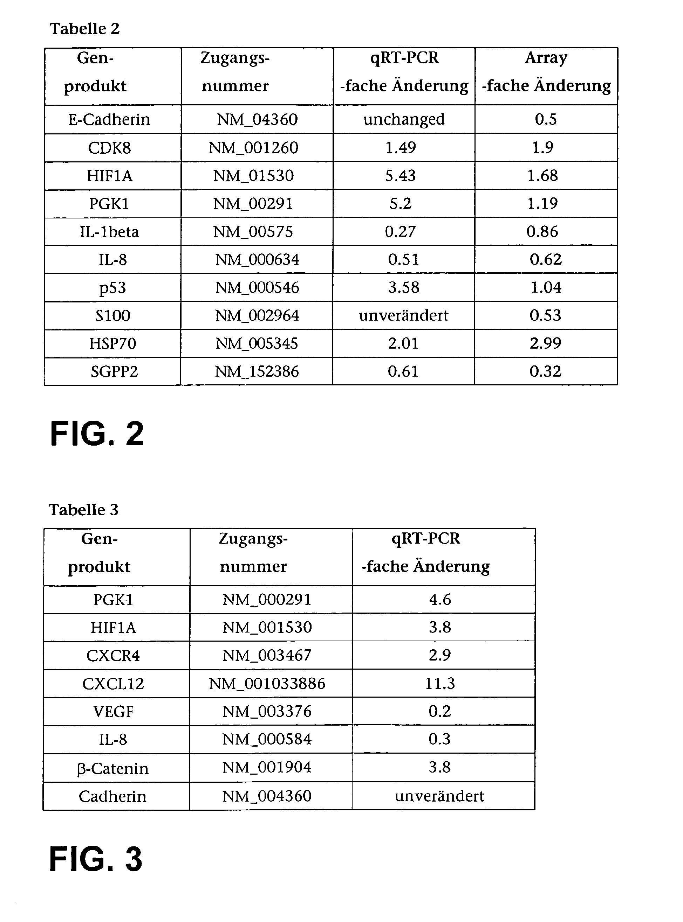

Die Genexpressionsanalyse wurde unter Verwendung eines extra angefertigten Oligo-Microarrays durchgeführt. Eine Reihe von Genen wurde identifiziert, die hoch- oder herunterreguliert waren, im Vergleich von diffusen Primär-Magentumorproben mit und ohne Peritonealkarzinosen. Nach Normalisierung wurde der t-Test angewandt, um differentiell exprimierte Gene in Peritonealkarzinosenproben vorhersagen zu können. Bei einem Signifikanz-Level von p<0,05 wurden 57 Gene als differenziert reguliert identifiziert, von welchen 25 in den Peritonealkarzinosenproben hochreguliert und 32 Gene herunterreguliert waren. Um dieses Ergebnisse zu validieren, wurden 10 Gene für eine Evaluierung mit der quantitativen Real Time PCR mit gepoolten Proben-RNA ausgewählt. Für sämtliche dieser Gene konnte deren Expression, die auf den Microarrays beobachtet wurde, bestätigt werden, mit Ausnahme von E-Cadherin und S100. Die Ergebnisse sind in Tabelle 2 der Fig. 2 gezeigt. Ein signifikanter p-Wert auf dem Microarray und der mRNA-Überexpression mittels quantitativer Real Time PCR (gepoolte Proben) wurde für PGK1 (p-Wert = 0,015 auf dem Microarray, 5,2-fach in der Real Time PCR in Primärtumorproben mit bekannter Peritonealkarzinose beobachtet. Basierend auf diesem Ergebnis wurden weitere Gene unter Verwendung der quantitativen Real Time PCR einzelner Proben (nicht gepoolt) untersucht. Diese Gene waren PGK1, HIF-la, CXCR4, CXCL12, beta-Catenin, VEGF und IL-8. Eine spezifische mRNA-Überexpression in diffusen Primärtumoren von Magenkrebs mit Peritonealkarzinose wurde für PGK1 (4,6-fach), HIF-1α (3,8-fach), CXCR4 (2,9-fach), CXCL12 (11,3-fach) und beta-Catenin (3,8-fach) detektiert, wohingegen eine spezifische mRNA-Supprimierung von VEGF (5-fach) in diffusen Primärtumoren von Magenkrebs mit Peritonealkarzinose detektiert wurde. Für IL-8 wurde ein großer Bereich von Expressionsunterschieden unter den Patienten beobachtet (siehe Fig. 1, äußerster rechter Plot der unteren Reihe). Dennoch deuten sowohl die Array-Daten als auch die quantitativen Real-Time-PCR-Daten aus der gepoolten RNA auf eine zweifache Herunterregulierung in Primärtumoren von Magenkrebsgeweben, die Peritonealkarzinose entwickelten, mit Primärtumoren ohne Peritonealkarzinose. Ein Vergleich von Primärtumoren mit Peritonealkarzinosen mit Primärtumoren ohne Peritonealkarzinosen konnte keine unterschiedliche Cadherin-mRNA beobachtet werden. Die Ergebnisse der quantitativen Real Time PCR einzelner Proben sind in der Tabelle 3 der Fig. 3 gezeigt.

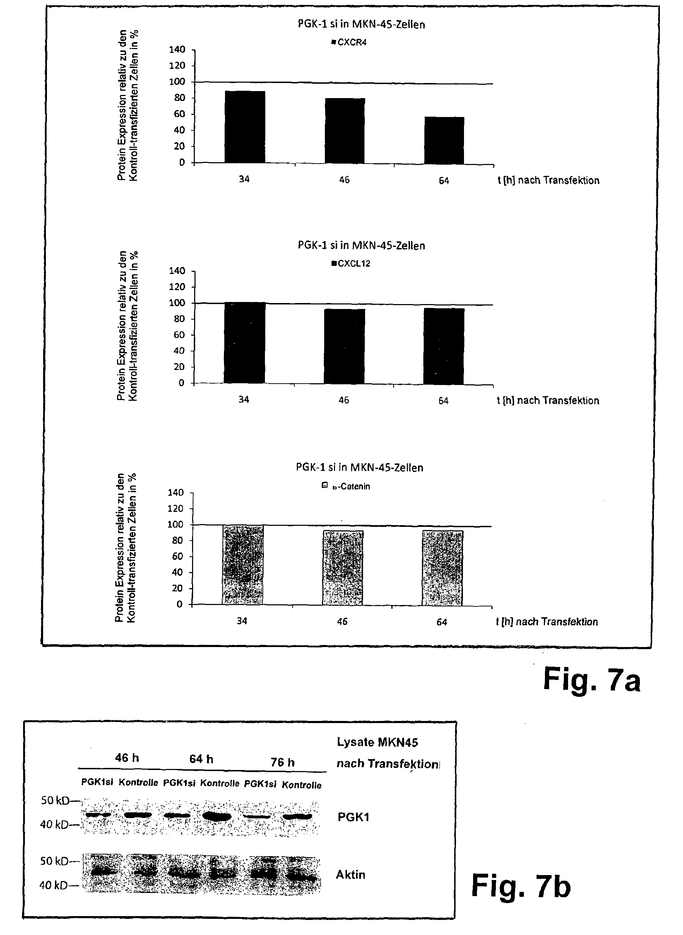

Mittels der Versuche zum Genedownsilencing und der Überexpression konnte gezeigt werden, dass PGK1 einen wichtigen Einfluss auf die Regulation der Signalmoleküle CXCR4, CXCL12 beta-Catenin und VEGF hat, wie auch *vice versa.* Diese Ergebnisse bestätigen somit die auf Expressionsebene generierten Daten, so dass der Einfluss von PGK1 auf seine Signalmoleküle weiter bestätigt und ein Regulationsmechanismus auf Expressions- sowie Proteinebene gezeigt werden konnte (siehe Fig. 7a/b). Aus den Ergebnissen zur Überexpression von PGK1 (Transfektion von PGK1) (siehe Fig. 8) ergibt sich, dass PGK1 mRNA sowie CXCR4 mRNA im Vergleich zur Kontrolle ohne Transfektion hochreguliert waren (siehe Fig. 8 links oben). Ferner ist aus den FACS-Ergebnissen von CXCR4, b-Catenin und CXCL12 auf Proteinebene zu erkennen, dass diese Gene in den Zellen mit PGK1-Transfektion auch wieder im Vergleich zu den Kontrollen hochreguliert sind (siehe Fig. 8 rechts oben). Auch konnte gezeigt werden, dass PGK1 auch auf Proteinebene in den transfizierten Zellen im Vergleich zu den Kontrollen vermehrt vorhanden ist (siehe Fig. 8, unten).

Die Untersuchung zur Invasivität der Zellen mit und ohne PGK1-Überexpression zeigte, dass von den Magenkarzinomzellen MKN45 ohne PGK1-Überexpression nur 2 Zellen invasiv in das Gel eingedrungen sind (siehe Fig. 9, links). Hingegen waren von den Magenkarzinomzellen MKN45 mit PGK1-Überexpression ca. 100 Zellen invasiv in das Gel eingedrungen (siehe Fig. 9, rechts).

Diese 50-fach gesteigerte Invasivität der Magenkarzinomzellen durch eine PGK1-Überexpression im Vergleich zu den Magenkarzinomzellen ohne eine PGK1-Überexpression zeigt den malignitätssteigernden Einfluss von PGK1 und seinen Signalmolekülen hinsichtlich Tumorgenese und der Peritonealkarzinose.

### Diskussion

In den oben genannten Versuchen wurde die differentielle Expression von Genen in Proben von Patienten mit diffusen Magenkrebs mit und ohne Peritonealkarzinosen unter Verwendung von Oligonucleotid-Microarrays und quantitativer Real Time PCR untersucht. Dies stellt nach Erkenntnissen des Erfinders die erste Studie dar, die eine hohe spezifische Überexprimierung von PGK1 mRNA in diffusem primären Magenkrebs mit histologisch nachgewiesener Peritonealkarzinose zeigt.

Abgesehen von seiner Funktion als glycolytisches Enzym, spielt PGK1 auch eine wichtige Rolle bei malignen Tumoren, wie bspw. Brustkrebs, Gebärmutter- und Pankreaskrebs, jedoch bisher nicht bei Magenkrebs. Die vorliegenden Ergebnisse zeigen, dass eine PGK1-Überexprimierung die Disseminierung in das Peritoneum fördern könnte.

HIF-1α reguliert bekanntermaßen die Expression von PGK1. Die Ausschaltung von HIF-1α stoppt die Induktion von PGK1, was bestätigt, dass PGK1 ein Downstream-Target von HIF-1α ist. Die vorliegenden Ergebnisse zeigen eine Überexprimierung von HIF-1α mRNA in Magenkrebs-Primärtumoren mit Peritonealkarzinosen, was in einem eindeutigem Zusammenhang steht mit den gewonnenen Expressionswerten von PGK1.

Die vorliegenden Ergebnisse zeigten ferner einen bedeutenden Anstieg in CXCR4-und CXCL12 mRNA, sowie eine starke Supprimierung von VEGF und IL-8 mRNA in Magenkrebsgewebe mit Peritonealkarzinosen. Damit sind auch die vorliegenden Ergebnisse die ersten, die eine starke Verknüpfung der PGK1-Regulierung und der CXCR4-/CXCL12-Achse in diffusen Primärmagenkrebs mit bekannter Peritonealkarzinosen zeigen.

Die vorliegenden Ergebnisse zeigten ferner eine Überexpression von β-Catenin mRNA in Primärtumoren mit Peritonealkarzinose. Diese Ergebnisse stehen im Gegensatz zu Berichten, bei denen Prostatakrebs-Zelllinien untersucht wurden, und bei denen hohe Level an PGK1 zu einer herunterregulierten Expression von β-Catenin führten.

Bis zum heutigen Tage existieren keine echten Diagnose-Marker, mit welchen vorhergesagt werden könnte, dass sich ein primärer Magentumor peritoneal streut. Es gibt zwar einige quantitative Prognose-Marker, die als Leitlinien bei der Auswahl der Behandlung dienen, um eine maximal wirksame Therapie zu erreichen, die jedoch nicht für die Vorhersage einer Streuung eingesetzt werden.

Die sehr eindeutige differentielle Expression von PGK1, HIF-1α, CXCR4, CXCL12, beta-Catenin und VEGF ist hochspezifisch. Daher können diese Moleküle als herausragende Marker, entweder alleine oder in Kombination miteinander verwendet werden, um Primärtumoren zu detektieren, die mit hoher Wahrscheinlichkeit eine peritoneale Streuung entwickeln.

## Patentansprüche

1. Verwendung von Phosphoglycerat-Kinase 1 (PGK1), oder Fragmenten davon, als diagnostischer Marker in einem in-vitro Verfahren zur Vorhersage oder Diagnose von Peritonealkarzinosen, wobei der Expressionslevel von PGK1, oder Fragmenten davon, als Marker dient.

2. Verwendung nach Anspruch 1, **dadurch gekennzeichnet, dass** der Expressions- level von Phosphoglycerat-Kinase 1 (PGK1) in Kombination mit dem Expressionslevel von beta-Catenin, CXCR4 und/oder CXCL12 als diagnostischer Marker verwendet wird.

3. Verwendung nach Anspruch 1 oder 2, **dadurch gekennzeichnet, dass** eine Hochregulierung der Genexpression von Phosphoglycerat-Kinase 1 (PGK1), in Kombination mit CXCR4, CXCL12 und/oder beta-Catenin, untersucht wird.

4. Verwendung nach Anspruch 1 oder 2, **dadurch gekennzeichnet, dass** eine Hochregulierung der Proteinexpression von Phosphoglycerat-Kinase 1 (PGK1), in Kombination mit CXCR4, CXCL12 und/oder beta-Catenin, untersucht wird.

5. Verwendung nach einem der Ansprüche 1 oder 2, **dadurch gekennzeichnet, dass** eine Sequenz eingesetzt wird, die zumindest eine der Sequenzen mit den SEQ ID-Nr. 1 bis 6, 15 und 16 aufweist, zur Identifizierung der diagnostischen Marker.

6. Verfahren zur Vorhersage der Entwicklung oder Diagnose von Peritonealkarzinosen, **dadurch gekennzeichnet, dass** es den *In-vitro*-Nachweis einer erhöhten Gen- oder Proteinexpression von Phosphoglycerat-Kinase 1 (PGK1) in einer biologischen Probe umfasst.

7. Verfahren nach Anspruch 6, **dadurch gekennzeichnet, dass** es die folgenden Schritte aufweist:
(a) Bereitstellen von biologischen Proben von zu untersuchenden Patienten, die an einem Tumor erkrankt sind,
(b) *In-vitro*-Nachweis einer erhöhten Gen- oder Proteinexpression von Phosphoglycerat-Kinase 1 (PGK1) in biologischen Proben im Vergleich zu Kontrollen und/oder Standards.

8. Verfahren nach Anspruch 6 oder 7, **dadurch gekennzeichnet, dass** es die folgenden Schritte aufweist:
(a) Bereitstellen von biologischen Proben von zu untersuchenden Patienten, die an einem Tumor erkrankt sind,
(b) In-vitro-Nachweis einer erhöhten Gen- oder Proteinexpression von Phosphoglycerat-Kinase 1 (PGK1) in Kombination mit CXCR4, CXCL12 oder beta-Catenin, in den biologischen Proben im Vergleich zu Kontrollen und/oder Standards.

9. Verfahren nach einem der Ansprüche 6 bis 8, **dadurch gekennzeichnet, dass** die biologische Probe eine flüssige oder feste Körperprobe eines Patienten ist.

10. Verfahren nach Anspruch 9, **dadurch gekennzeichnet, dass** die Körperprobe ausgewählt ist aus Blut, Serum, Plasma, Urin, Gewebe, Knochen, Knorpel, Lymphflüssigkeit, und/oder aus einem Organ ist.

11. Verfahren nach Anspruch 9 oder 10, **dadurch gekennzeichnet, dass** die Körperprobe eine Tumorprobe ist.

12. Wirkstoff, der die Aktivität und/oder Expression von Phosphoglycerat-Kinase 1 (PGK1) hemmt, zur Verwendung bei der Behandlung und/oder Prävention von Peritonealkarzinosen, wobei der Wirkstoff ausgewählt ist aus siRNA oder einem antisense-Molekül, das PGK1 bindet.

13. Wirkstoff zur Verwendung nach Anspruch 12, **dadurch gekennzeichnet, dass** der Wirkstoff, der die Aktivität und/oder Expression von Phosphoglycerat-Kinase 1 (PGK1) hemmt, in Kombination mit einem aus siRNA und einem antisense Molekül ausgewählten Wirkstoff, der die Aktivität und/oder Expression von beta-Catenin, CXCR4 und/oder CXCL12, hemmt, eingesetzt wird.

14. Wirkstoff zur Verwendung nach Anspruch 12 oder 13, **dadurch gekennzeichnet, dass** der Wirkstoff, der die Aktivität und/oder Expression von Phosphoglycerat-Kinase 1 (PGK1) hemmt, in Kombination mit zumindest einem Chemotherapeutikum eingesetzt wird.

15. Pharmazeutische Zusammensetzung zur Verwendung bei der Behandlung und/oder Prävention von Peritonealkarzinosen umfassend einen Wirkstoff, der die Aktivität und/oder die Expression von Phosphoglycerat-Kinase 1 (PGK1) hemmt, sowie ggf. zumindest einen pharmazeutisch akzeptierbaren Träger, wobei der Wirkstoff ausgewählt ist aus siRNA oder einem antisense-Molekül, das PGK1 bindet.

16. Pharmazeutische Zusammensetzung zur Verwendung nach Anspruch 15, **dadurch gekennzeichnet, dass** sie ferner zumindest einen aus siRNA und einem antisense-Molekül ausgewählten Wirkstoff umfasst, der die Aktivität und/oder Expression von beta-Catenin, CXCR4 und/oder CXCL12 hemmt, und ggf. zumindest einen pharmazeutisch akzeptierbaren Träger.

## Claims

1. The use of phosphoglycerate kinase 1 (PGK1), or fragments thereof, as a diagnostic marker in an in vitro method for the prognosis or diagnosis of peritoneal carcinoses, wherein the level of expression of PGK1, or fragments thereof, is used as marker.

2. The use as claimed in claim 1, **characterized in that** the level of expression of phosphoglycerate kinase 1 (PGK1) is used in combination with level of the expression of β-catenin, CXCR4, and/or CXCL12 as diagnostic markers.

3. The use as claimed in claims 1 or 2, **characterized in that** an upregulation of the gene expression of phosphoglycerate kinase 1 (PGK1), in combination with CXCR4, CXCL12, and/or β-catenin is investigated.

4. The use as claimed in claims 1 or 2, **characterized in that** upregulation of the protein expression of phosphoglycerate kinase 1 (PGK1), in combination with CXCR4, CXCL12, and/or β-catenin is investigated.

5. The use as claimed in claims 1 or 2, **characterized in that** a sequence is used that comprises at least one of the sequences having SEQ ID Nos. 1 through 6, 15, and 16 for identifying the diagnostic markers.

6. A method for the prognosis of development or diagnosis of peritoneal carcinoses, **characterized by** comprising the *in vitro* detection of increased gene or protein expression of phosphoglycerate kinase 1 (PGK1) in a biological sample.

7. The method as claimed in claim 6, **characterized by** comprising the following steps:
a) Provision of biological samples from tumor patients to be examined.
b) *In vitro* detection of increased gene or protein expression of phosphoglycerate kinase 1 (PGK1) in biological samples compared to controls and/or standards.

8. The method as claimed in claims 6 or 7, **characterized by** comprising the following steps:
a) Provision of biological samples from tumor patients to be examined.
b) *In vitro* detection of increased gene or protein expression of phosphoglycerate kinase 1 (PGK1) in combination with CXCR4, CXCL12, or β-catenin in said biological samples compared to controls and/or standards.

9. The method as claimed in one of claims 6 through 8, **characterized in that** the biological sample is a fluid or solid body sample from a patient.

10. The method as claimed in claim 9, **characterized in that** the body sample is selected from blood, serum, plasma, urine, tissue, bone, cartilage, lymph, and/or is from an organ.

11. The method as claimed in claims 9 or 10, **characterized in that** the body sample is a tumor sample.

12. An active component that inhibits the activity and/or expression of phosphoglycerate kinase 1 (PGK1) for use in the treatment and/or prevention of peritoneal carcinoses, wherein the active component is selected from siRNA or from an antisense-molecule, which binds to PGK1.

13. The active component for use as claimed in claim 12, **characterized in that** the active component that inhibits the activity and/or expression of phosphoglycerate kinase 1 (PGK1), is used in combination with an active component that is selected from siRNA or an antisense-molecule and that inhibits the activity and/or expression of or β-catenin, CXCR4, and/or CXCL12.

14. The active component as claimed in claim 12 or 13, **characterized in that** the active component, which inhibits the activity and/or expression of phosphoglycerate kinase 1 (PGK1), is used in combination with at least one chemotherapeutic agent.

15. A pharmaceutical composition for use in the treatment and/or prevention of peritoneal carcinoses, comprising an active component that inhibits the activity and/or expression of phosphoglycerate kinase 1 (PGK1) and optionally at least one pharmaceutically acceptable carrier, wherein the active component is selected from siRNA or from an antisense-molecule, which binds to PGK1.

16. The pharmaceutical composition for use as claimed in claim 15, **characterized in that** it further comprises at least one active component that is selected from siRNA and an antisense-molecule and that inhibits the activity and/or expression of β-catenin, CXCR4, and/or CXCL12, and optionally, at least one pharmaceutically acceptable carrier.

## Revendications

1. Utilisation de phosphoglycérate-kinase 1 (PGK1) ou de fragments de cette dernière, en tant que marqueur diagnostique dans un procédé *in vitro* de prédiction ou de diagnostic de carcinoses péritonéales, le niveau d'expression de PGK1 ou de fragments de cette dernière servant de marqueur.

2. Utilisation selon la revendication 1, **caractérisée en ce qu'**on utilise en tant que marqueur diagnostique le niveau d'expression de la phosphoglycérate-kinase 1 (PGK1) en combinaison avec le niveau d'expression de la bêta-caténine, de CXCR4 et/ou de CXCL12.

3. Utilisation selon la revendication 1 ou 2, **caractérisée en ce qu'**on examine une régulation positive de l'expression génique de la phosphoglycérate-kinase 1 (PGK1), en combinaison avec CXCR4, CXCL12 et/ou la bêta-caténine.

4. Utilisation selon la revendication 1 ou 2, **caractérisée en ce qu'**on examine une régulation positive de l'expression protéique de la phosphoglycérate-kinase 1 (PGK1), en combinaison avec CXCR4, CXCL12 et/ou la bêta-caténine.

5. Utilisation selon l'une des revendications 1 ou 2, **caractérisée en ce qu'**on utilise pour identifier les marqueurs diagnostiques une séquence qui présente l'une des séquences SEQ ID NO:1 à 6, 15 et 16.

6. Procédé pour la prédiction du développement ou le diagnostic de carcinoses péritonéales, **caractérisé en ce qu'**il comprend la détection *in vitro* d'une expression génique ou protéique accrue de phosphoglycérate-kinase 1 (PGK1) dans un échantillon biologique.

7. Procédé selon la revendication 6, **caractérisé en ce qu'**il comprend les étapes suivantes :
(a) mise à disposition d'échantillons biologiques de patients à examiner, qui présentent une tumeur,
(b) détection in vitro d'une expression génique ou protéique accrue de phosphoglycérate-kinase 1 (PGK1) dans des échantillons biologiques, par comparaison avec des témoins et/ou des étalons.

8. Procédé selon la revendication 6 ou 7, **caractérisé en ce qu'**il comprend les étapes suivantes :
(a) mise à disposition d'échantillons biologiques de patients à examiner, qui présentent une tumeur,
(b) détection in vitro d'une expression génique ou protéique accrue de phosphoglycérate-kinase 1 (PGK1) en combinaison avec CXCR4, CXCL12 ou la bêta-caténine, dans les échantillons biologiques, par comparaison avec des témoins et/ou des étalons.

9. Procédé selon l'une des revendications 6 à 8, **caractérisé en ce que** l'échantillon biologique est un échantillon corporel liquide ou solide d'un patient.

10. Procédé selon la revendication 9, **caractérisé en ce que** l'échantillon corporel est choisi parmi le sang, le sérum, le plasma, l'urine, les tissus, les os, le cartilage, la lymphe et/ou un organe.

11. Procédé selon la revendication 9 ou 10, **caractérisé en ce que** l'échantillon corporel est un échantillon tumoral.

12. Principe actif qui inhibe l'activité et/ou l'expression de la phosphoglycérate-kinase 1 (PGK1) pour une utilisation lors du traitement et/ou la prévention de carcinoses péritonéales, le principe actif étant choisi parmi le pARNi ou une molécule antisens qui se lie à PGK1.

13. Principe actif pour une utilisation selon la revendication 12, **caractérisé en ce que** le principe actif qui inhibe l'activité et/ou l'expression de la phosphoglycérate-kinase 1 (PGK1) est utilisé en combinaison avec un principe actif choisi parmi le pARNi et une molécule antisens, qui inhibe l'activité et/ou l'expression de la bêta-caténine, du CXCR4 et/ou du CXCL12.

14. Principe actif pour une utilisation selon la revendication 12 ou 13, **caractérisé en ce que** le principe actif qui inhibe l'activité et/ou l'expression de la phosphoglycératekinase 1 (PGK1) est utilisé en combinaison avec au moins un agent chimiothérapeutique.

15. Composition pharmaceutique pour une utilisation lors du traitement et/ou la prévention de carcinoses péritonéales, comprenant un principe actif qui inhibe l'activité et/ou l'expression de la phosphoglycérate-kinase 1 (PGK1), ainsi qu'éventuellement au moins un support pharmaceutiquement acceptable, le principe actif étant choisi parmi le pARNi ou une molécule antisens qui se lie au PGK1.

16. Composition pharmaceutique pour une utilisation selon la revendication 15, **caractérisée en ce qu'**elle comprend en outre au moins un principe actif choisi parmi le pARNi et une molécule antisens, qui inhibe l'activité et/ou l'expression de la bêtacaténine, du CXCR4 et/ou du CXCL12, et éventuellement au moins un support pharmaceutiquement acceptable.
